# EUROPEAN PATENT APPLICATION

(11) **EP 2 250 911 A1**
(43) Date of publication of application: **17.11.2010**
(21) Application number: 10007443.4
(22) Date of filing: 15.10.2004
(51) Int. Cl.: A23L 1/29, A61K 36/899, A61P 3/04, A23L 1/30, A23L 1/308

(54) **Weight-loss supplement**

(30) Priority: 17.10.2003 US 512344 P; 08.01.2004 US 535471 P
(62) Divisional of application: 04789736.8
(71) Applicant: Diet Formulations Ltd., Oakville ON L6M 0H4 (CA)
(72) Inventor: Jacobs, Ira, Thornhill, Ontario L3T 6T2 (CA); Gardiner, Paul T., Mississauga, Ontario L4W SS2 (CA); Molino, Michael, Woodbridge, Ontario l4H 2W1 (CA)
(74) Representative: Kiriczi, Svenja

(57) **Abstract**

The invention is directed to a weight-loss supplement comprising a satiety-promoting ingredient that includes a dietary fiber comprising oat bran and a thermogenic ingredient selected from the group consisting of caffeine, catechin-polyphenol and combinations thereof.

## Description

### RELATED APPLICATIONS

This application is based on and claims priority to Applicants' co-pending U.S. Provisional Patent Application Serial No. 60/512,344, filed on October 17, 2003, and U.S. Provisional Patent Application Serial No. 60/535,471, filed on January 8, 2004. The disclosures of these provisional applications are incorporated herein by reference as fully as if set forth in their entirety.

### FIELD OF THE INVENTION

This application relates to a weight loss supplement and more particularly to a weight loss supplement that includes a satiety-promoting ingredient and a thermogenic ingredient.

### BACKGROUND

The maintenance of a desired body weight in mammals, including humans, is a direct function of the balance of nutritional caloric consumption on the one hand, and caloric expenditure, also known as metabolic rate, on the other.

Primary factors known to affect caloric consumption are appetite or satiety, and socio-cultural factors which affect eating and feeding behaviors. The feeling of fullness in the stomach is thought to decrease appetite and increase satiety. Therefore, the normal response to eating is that the desire to eat more decreases as a function of the amount of food consumed.

There is a direct relationship between changes in many factors and changes in metabolic rate. The following are examples of such factors: ambient temperature, body size, the proportion of body weight consisting of muscle tissue, the level of physical exertion, genetic predisposition to have a naturally occurring higher or lower resting metabolic rate. Pharmacological, nutritional and physiological manipulations can also be used to manipulate metabolic rate. According to Bray (Bray, GA, Nutrition. 2000 Oct; 16(10):953-60, incorporated herein by reference in its entirety, hereinafter "Bray") drugs to treat obesity can be divided into three groups: those that reduce food intake; those that alter metabolism; and those that increase thermogenesis. The ingestion of a combination of caffeine with ephedrine is an example of a pharmacological treatment which has been shown to be an effective anti-obesity drug treatment in humans and the mechanism of action has been attributed to increased metabolic rate (Toubro, S., A. Astrup, L. Breum, and F. Quaade. Safety and efficacy of long-term treatment with ephedrine, caffeine and an ephedrine/caffeine.mixture. Int. J. Obes. Relat. Metab Disord. 17:S69-72, 1993.). The very act of eating a meal induces an increase in metabolic rate, called the "Thermic Effect of Food" which has been attributed to the increased physiological functions necessary to digest the food Even prolonged cold exposure has been suggested as being an effective, albeit unpleasant weight-loss treatment because of the increased metabolic rate associated with shivering.

According to Dulloo et al., Am. J. Clin. Nutr. 1999, 70:1040-5 ("Dulloo et al."), which is incorporated herein by reference in its entirety, a green tea extract stimulates tissue thermogenesis to an extent which is much greater than can be attributed to its caffeine content per se. Dulloo et al. hypothesized that the thermogenic properties of green tea could reside primarily in an interaction between the high content in catechin-polyphenols and caffeine with sympathetically released noradrenaline (NA). Dulloo et al. hypothesized that green tea extract, via its catechin-polyphenols and caffeine content, was effective in stimulating thermogenesis by relieving inhibition at different control points along the NA-cAMP axis. Thus, Dulloo et al. proposedia synergistic interaction between catechin-polyphenols and caffeine to augment and prolong sympathetic stimulation of thermogenesis; he indicated that this interaction could be of value in assisting the management of obesity.

According to Astrup et al., Int J Obes Relat Metab Disord. 1992 Apr; 16(4):269-77 ("Astrup et al."), which is incorporated herein by reference in its entirety, the sympathomimetic agent ephedrine has potent thermogenic and anti-obesity properties in rodents. Astrup et al. state that in rodents the effect is markedly enhanced by caffeine, while caffeine given alone has no effect. Astrup et al. also state that in analogy with animal studies, the ephedrine/caffeine combination is effective in humans, while caffeine and ephedrine separately are ineffective for the treatment of human obesity.

U.S. Patent No. 5,422,352; ("the '352 patent"), which is incorporated herein by reference in its entirety, refers to a method for reducing the weight of a human and a method for reducing the adipose tissue mass/lean body mass ratio of a human or a domestic animal. The `352 patent further states that in each method, an effective dose of a combination of ephedrine and caffeine are administered in a weight ratio of about 1:12, respectively, calculated on the amount of ephedrine in the form of the free base.

U.S. Patent No. 6,475,530 ("the '530 patent"), which is incorporated herein by reference in its entirety, is directed to compositions of noradrenaline stimulating agents such as ephedra, citrus aurantium, or other botanical source of adrenaline stimulating alkaloids. The '530 patent refers to methods and compositions for producing weight loss in a mammal by administration of a composition containing a weight loss effective amount of a noradrenaline stimulating compound such as ephedrine, mahuang (a plant source of ephedrine alkaloids), citrus aurantium

(bitter orange), synephrine, norephedrine, psuedophedrine, a methylxanthine, such as caffeine or guarana, and a botanical COX inhibitor such as resveratrol polygonum cuspidatum, scutellaria baicalensis, turmeric, curcumin, rosemary, green tea, ocimum sanctum (holy basil), or ginger instead of an NSAID such as aspirin, and optionally a free fatty acid reducing compound. The specification states that a thermogenic formula is coupled with a growth hormone stimulating formulation containing L-arginine or L-omithine, L-lysine, and a free fatty acid reducing agent such as nicotinic acid. According to the patent, the thermogenic formula would preferably be administered in the daytime, and the growth hormone producing formula at nighttime. As referred to in the patent, the two compositions form a system of AM and PM weight loss strategy for the therapeutic intervention of obesity.

U.S. Patent No. 6,383,482 ("the '482 patent"), which is incorporated herein by reference in its entirety, refers to a formulation for weight loss containing green tea extract, hydroxycitric acid, 5-hydroxytryptophan, glucomannan, chromium picolinate, and Lactobacillus acidophilus. The '482 patent states that the formulation suppresses appetite and helps to burn fat.

According to Di Buono et al., Canadian Journal of Diabetes, 26: 309, 2002 ("Di Buono"), the ability of soluble fiber to lower postprandial blood glucose is related to its viscosity which results in slower nutrient absorption. Moreover, Di Buono states that the viscosity of a glucomannan: xanthan mixture increases dramatically over the viscosity of glucomannan alone.

### SUMMARY OF THE INVENTION

The present invention, in particular according to one embodiment thereof, provides for a weight-loss supplement that comprises
(a) a satiety-promoting ingredient that includes a mixture of dietary fibers comprising glucomannan and a gum and/or oat bran, and preferably a gum, and;
(b) a thermogenic ingredient selected from the group consisting of caffeine, catechin-polyphenol and combinations thereof.

According to a further embodiment of the invention the gum is selected from the group consisting of xanthan gum, guar gum, cargeenan, and locust bean gum. According to a further embodiment of the invention the gum is xanthan gum. According to a further embodiment of the invention the gum comprises a mixture of gums. According to a further embodiment of the invention the mixture of gums comprises at least one gum selected from the group consisting of xanthan gum, guar gum, cargeenan, and locust bean gum. According to a further embodiment of the invention a serving comprises from about 0.1 g to about 10 g of the weight-loss supplement. According to a further embodiment of the invention a serving comprises from about 1 g to about 5 g of the weight-loss supplement. According to a further embodiment of the invention a serving comprises about 3.0 g of the weight-loss supplement. According to a further embodiment of the invention the glucomannan and/or oat bran is present in an amount from about 0.1 g to about 5 g per serving. According to a further embodiment of the invention the glucomannan and/or oat bran is present in an amount from about 1 g to about 3 g per serving. According to a further embodiment of the invention the glucomannan and/or oat bran is present in an amount of about 2 g. According to a further embodiment of the invention the gum is present in an amount from about 0.1 g to about 5 g per serving. According to a further embodiment of the invention the gum is present in an amount from about 0.3 g to about 3 g per serving. According to a further embodiment of the invention the gum is present in an amount of about 0.5 g per serving. According to a further embodiment of the invention glucomannan and/or oat bran and the gum have a weight ratio from about 1:1 to about 5:1. According to a further embodiment of the invention glucomannan and/or oat bran and the gum have a weight ratio of about 4:1. According to a further embodiment of the invention a serving comprises glucomannan and/or oat bran at about 1.8 g and xanthan gum at about 440 mg per serving. According to a further embodiment of the invention the thermogenic ingredient comprises a tea of camellia sinensis. According to a further embodiment of the invention the thermogenic ingredient comprises a green tea that is the unfermented camellia sinensis leaf. According to a further embodiment of the invention the thermogenic ingredient comprises an enriched green tea extract. According to a further embodiment of the invention a serving comprises from about 25 mg to about 1000 mg of caffeine. According to a further embodiment of the invention a serving comprises from about 50 mg to about 250 mg of caffeine. According to a further embodiment of the invention a serving comprises about 200 mg of caffeine. According to a further embodiment of the invention the thermogenic ingredient comprises catechin-polyphenol selected from the group consisting of epigallocatechin-gallate, epicatechin-gallate, epicatechin, catechin and epigallocatechin. According to a further embodiment of the invention the serving comprises from about 1 to about 1000 mg of a catechin-polyphenol. According to a further embodiment of the invention a serving comprises from about 75 to about 500 mg of catechin-polyphenol. According to a further embodiment of the invention a serving comprises about 300 mg of EGCG. According to a further embodiment of the invention the composition further comprises at least one ingredient selected from the group consisting of a chromium chelate, L-carnitine, Guarana, Yerba Mate, White Willow Bark, Garcinia Cambogia, Alpha Lipoic Acid, Ginseng, Gymnema Sylvestre and Cellulose. According to a further embodiment of the invention the chromium chelate is selected from the group consisting of chromium picolinate and chromium poly-nicotinate.

The present invention, in particular according to one embodiment thereof, provides for a weight-loss supplement that comprises glucomannanGlucomannan and/or oat bran as well as xanthan gum, Green Tea, Anhydrous Caffeine, Chromium Polynicotinate, Guarana, Yerba Mate and Alpha Lipoic Acid.

The present invention, in particular according to one embodiment thereof, provides for a weight-loss supplement that comprises glucomannan and/or oat bran in a quantity of about 500 mg to about 5000 mg as well as Xanthan gum in a quantity of about 100 mg to about 1000 mg, Green Tea in a quantity of about 100 mg to about 1000 mg, Anhydrous Caffeine in a quantity of about 10 mg to about 600 mg, Chromium Polynicotinate, in a quantity of about 10 mcg to about 500 mcg, Guarana in a quantity of about 50 mg to about 1000 mg, Yerba Mate, in a quantity of about 1 mg to about 2000 mg, Alpha Lipoic Acid, in a quantity of about 1 mg to about 1000 mg.

The present invention, in particular according to one embodiment thereof, provides for a weight-loss supplement that comprises glucomannan in a quantity of about 1.76 mg, xanthan gum in a quantity of about 440 mg, Green Tea (45% EGCG) 500 mg, Green Tea (15% EGCG, 40% Caffeine) 500 mg, Chromium polynicotinate in a quantity of about 16. 5 mcg, Guarana in a quantity of about 10 mg, Yerba Mate in a quantity of about 10 mg and Alpha Lipoic Acid in a quantity of about 100 mg.

The present invention, in particular according to one embodiment thereof, provides for a weight-loss supplement that comprises Oat Bran in a quantity of about 1. 8 mg, Xanthan gum in a quantity of about 440 mg, Green Tea (45% EGCG) in a quantity of about 500 mg, Green Tea (15% EGCG, 40% Caffeine) in a quantity of about 500 mg, Guarana in a quantity of about 10 mg, Yerba Mate in a quantity of about 10 mg and Alpha Lipoic Acid in a quantity of about 100 mg.

The present invention, in particular according to one embodiment thereof, provides for a method for inducing, in particular non-therapeutic, weight loss that comprises the step of administering an effective amount of a weight-loss supplement that comprises a satiety-promoting ingredient that includes a dietary fiber and a thermogenic ingredient selected from the group consisting of caffeine, catechin-polyphenol and combinations thereof.

According to a further embodiment of the invention the weight-loss supplement is administered about ½ 1 hour before meals. According to a further embodiment of the invention the weight-loss supplement is administered three times a day. According to a further embodiment of the invention wherein the weight-loss supplement is administered three times a day for a period of at least about a week. According to a further embodiment of the invention the weight-loss supplement is administered three times a day for a period of about at least 4 weeks. According to a further embodiment of the invention the weight-loss supplement is administered three times a day for a period of about at least 8 weeks. According to a further embodiment of the invention the dietary fiber comprises one of a mixture of glucomannan and a gum and oat bran. According to a further embodiment of the invention the gum is selected from the group consisting of xanthan gum, guar gum, cargeenan, and locust bean gum. According to a further embodiment of the invention the gum is xanthan gum. According to a further embodiment of the invention the dietary fiber comprises a mixture of glucomannan and a blend of gums. According to a further embodiment of the invention the blend of gums comprises at least one compound selected from the group consisting of xanthan gum, guar gum, cargeenan, and locust bean gum. According to a further embodiment of the invention the composition is administered ½ to 1 hour before meals. According to a further embodiment of the invention the composition is administered three times a day. According to a further embodiment of the invention the composition is administered as a capsule, as a tablet, as a caplet or as a ready-to- eat dietary supplement or food bar.

The present invention, according to one embodiment thereof, provides for a weight-loss supplement that includes a satiety-promoting ingredient, and a thermogenic ingredient, wherein the satiety-promoting ingredient is a mixture of dietary fibers including glucomannan and a gum and the thermogenic ingredient is selected from the group consisting of caffeine, catechin-polyphenol and combinations thereof.

The present invention, according to one embodiment, provides for a supplement that may provide any one or more of the following benefits: help reduce and control appetite; help controls food cravings; help reduce hunger cravings; help induce feeling of fullness (satiety); promote and support weight loss; help reduce body mass index (BMI); help reduce waist and thigh measurements; increase metabolism; increase thermogenesis; increase daily caloric expenditure; support normal blood sugar levels; and increase energy.

The present invention, according to one embodiment, provides for a weight-loss supplement that includes Glucomannan, Xanthan gum. Green Tea - Dry Leaf, Anhydrous Caffeine, Guarana. Yerba Mate, Alpha Lipoic Acid, and Chromium Polynicotinate.

In one embodiment, the weight-loss supplement includes Konjac Glucomannan at about 1.76g (e.g., 1.672g of Glucomannan - 95% purity); Xanthan gum at about 440 mg; Green Tea - Dry Leaf (4% caffeine, 45% EGCG) at about 667 mg (26.66mg Caffeine - 300 mg EGCG); Anhydrous Caffeine at about 173 mg; Guarana at about 1 mg; Yerba Mate at about 1 mg; Alpha Lipoic Acid at about 1 mg; and Chromium Polynicotinate to provide about 300 mcg of Elemental Chromium

The present invention, according to another embodiment, provides for a weight-loss supplement that includes Glucomannan, Xanthan gum, Caffeine, Catechin-polyphenol, Chromium chelate, L-carnitine, Guarana, Yerba Mate, White Willow Bark, Garcinia Cambogia, Alpha Lipoic Acid, Gymnema Sylvestre and Ginseng.

The present invention, according to one embodiment, also provides for a weight-loss supplement that preferably includes Glucomannan, in a quantity of about 500 mg to about 5000 mg; Xanthan gum, in a quantity of about 100 mg to about 1000 mg; Caffeine, in a quantity of about 10 mg to about 600 mg; Catechin-polyphenol about 45 to about 400 mg; Chromium chelate, in a quantity of about 10 mcg to about 500 mcg, L-carnitine, in a quantity of about 1 mg to about 500 mg; Guarana, in a quantity of about 50 mg to about 1000mg; Yerba Mate, in a quantity of about 1 mg to about 2000 mg; White Willow Bark, in a quantity of about 1 mg to about 500 mg; Garcinia Cambogia, in a quantity of about 1 mg to about 5000 mg; Alpha Lipoic Acid, in a quantity of about 1 mg to about 1000 mg; Gymnema Sylvestre , in a quantity of about 1mg to about 1000mg; and Ginseng, in a quantity of about 50 mg to about 500 mg.

The present invention, according to one embodiment, also provides for a weight-loss supplement that preferably includes: Glucomannan, in a quantity of about 1.8 mg; Xanthan gum, in a quantity of about 440 mg; Enriched Green Tea (camellia sinensis) 200 mg; Chromium polynicotinate, in a quantity of about 0.300 mg of elemental chromium; L-carnitine, in a quantity of about 100 mg; Guarana, in a quantity of about 528 mg; Yerba Mate, in a quantity of about 200 mg; Alpha Lipoic Acid, in a quantity of about 200 mg; and, American Ginseng, in a quantity of about 330 mg. Alternatively, the caffeine is caffeine anhydrous USP grade, in a quantity of about 200mg.

The present invention, according to one embodiment, also provides for a weight-loss supplement that preferably includes: Glucomannan, in a quantity of about 1.8 mg; Xanthan gum, in a quantity of about 440 mg; Caffeine, in a quantity of about 200 mg; Catechin-polyphenol, in a quantity of about 300 mg; Chromium, in a quantity of about 0.3 mg; L-carnitine, in a quantity of about 1 mg; Guarana, in a quantity of about 1 mg; Yerba Mate, in a quantity of about 1 mg; White Willow Bark, in a quantity of about 1 mg; Garcinia Cambogia, in a quantity of about 1 ing; and Alpha Lipoic Acid, in a quantity of about 1 mg.

The present invention, according to one embodiment, also provides for a method for inducing weight loss that includes the steps of administering an effective amount of a weight-loss supplement that includes a satiety-promoting ingredient and a thermogenic ingredient, wherein the satiety-promoting ingredient is a dietary fiber and the thermogenic ingredient is selected from the group consisting of caffeine, catechin-polyphenol and combinations thereof.

### BRIEF DESCRIPTION OF THE DRAWINGS

Figure 1 is a diagram that illustrates a sample size for subjects who completed the study stratified by treatment group, in accordance with one embodiment of the present invention;
Figure 2 is a diagram that illustrates the percent distribution of males and females in each treatment group, in accordance with one embodiment of the present invention;
Figure 3 is a diagram that illustrates the mean (standard deviation) age in years for each treatment group, in accordance with one embodiment of the present invention;
Figure 4 is a diagram that illustrates the total weight loss (lbs.) after 2, 4, 6 and 8 weeks of follow-up, stratified by treatment group, in accordance with one embodiment of the present invention; and
Figure 5 is a diagram that illustrates the total reduction in body mass index (BMI) after 2, 4, 6 and 8 weeks of follow-up, stratified by treatment group, in accordance with one embodiment of the present invention.

### DETAILED DESCRIPTION

The present invention provides for a weight-loss supplement comprising: a satiety-promoting ingredient, and; a thermogenic ingredient, wherein the satiety-promoting ingredient is a mixture of dietary fibers comprising glucomannan and a gum and the thermogenic ingredient is selected from the group consisting of caffeine, catechin-polyphenol and combinations thereof.

We have found that a dietary fiber mixture has unexpectedly high satiety-promoting properties. The preferred dietary fiber mixture is a mixture of glucomannan and xanthan gum. Moreover, we have found when the dietary fiber mixture is combined with ingredients that increase energy expenditure, the resulting composition is unexpectedly effective for inducing weight loss. Preferably the ingredients that increase energy expenditure comprise a combination of catechins polyphenols and caffeine.

As used herein, the term "dietary fiber" may include the indigestible carbohydrate and carbohydrate-like components of foods that are found predominantly in plant cell walls. It includes, but is not limited to, cellulose, lignin, hemicelluloses, such as glucomannan, pentosans, gums, such as xanthan gum, and pectins. Those fibers that have colligative properties, such as gums, are referred to as soluble fibers. They are often used to provide viscosity and texture in processed foods, and have been linked to lowered serum cholesterol levels. Insoluble fibers, such as cereal brans and specialty flour ingredients, tend to cause a laxative effect when consumed in large quantities. Dietary fiber has become an important food additive owing to the link between high fiber intake and the lowering of serum cholesterol, the prevention of certain types of cancer, and the avoidance of digestive tract disease.

As used herein, the term "mixture of dietary fibers" refers to a mixture of two or more dietary fibers.

As used herein, the term "a satiety-inducing ingredient" is an ingredient that promotes a sense of having been fed or fullness. Satiety-inducing ingredient are preferably dietary fibers, such as glucomannan (konjac), various gums, such as xanthan gum, guar gum, locust bean gum, carageenan, and combinations thereof.

As used herein, the term "a thermogenic ingredient" refers to an ingredient, which increases energy expenditure, also known as metabolic rate. The certain thermogenic ingredient may also have a property of inducing satiety. Examples of thermogenic ingredients, which may both increase the metabolic rate and induce satiety, are certain catechin polyphenols.

As used herein, the term "an effective amount" refers to an amount effective for inducing weight loss when administered as a serving three times a day over a period of week(s). An effective amount of the weight-loss supplement is preferably from about 0.1 g to about 10 g of the weight-loss supplement per serving. More preferably, an effective amount of the composition comprises from about 1 g to about 5 g of the weight-loss supplement per serving. Most preferably, an effective amount of the composition comprises about 3.0 g of the weight-loss supplement.

As used herein, the term "essentially pure" preferably refers to a compound that is greater than about 90% pure. More preferably, "essentially pure" refers to a compound that is greater than about 95% pure. Most preferably, "essentially pure" refers to a compound that is greater than about 98% pure.

As used herein, the term "enriched" refers to a partially purified extract or composition from which undesirable impurities have been removed. The undesirable impurities may be a single compound or multiple compounds. Preferably, "enriched" refers to a composition in which at least 25% of the undesirable impurities have been removed. More preferably, "enriched" refers to a composition in which at least 50% of the undesirable impurities have been removed. Most preferably, "enriched" refers to a composition in which at least 75% of the undesirable impurities have been removed.

In a preferred embodiment the satiety-promoting ingredient is a dietary fiber. Preferably, the dietary fiber comprises a mixture of glucomannan and a gum. The gum is preferably selected from the group consisting of xanthan gum, guar gum, carageenan, and locust beam gum Most preferably the gum is xanthan gum. Alternatively, the dietary fiber may comprise a mixture of glucomannan and a blend of gums. Preferably the blend of gums comprises at least one compound selected from the group consisting of xanthan gum, guar gum, carageenan and locust bean gum.

Glucomannan, also known as konjak or konjak-mannan, is a highly viscous, water-soluble dietary fiber obtained by grinding the tuber root of the *Amorphophallus Konjac* (also known as *Proteinophallus Riven*) plant. Glucomannan is differentiated from other soluble fibers by the extraordinarily high viscosity of Glucomannan solutions which are able to form gels. Administration of 4-5 g of Glucomanan with meals, blended into fluid or mixed with food, slows carbohydrate absorption and dampens the postprandial insulin response by up to 50%. Controlled clinical studies document that Glucomannan can promote satiety and weight loss.

Xanthan gum is produced by industrial fermentation of a carbohydrate under aerobic conditions by culturing the bacterium Xanthomonas campestris. It is unique in that it gives high viscosity solutions at low concentrations, exhibits little change in viscosity with variation in temperature, and is stable over a wide pH range. The greatest usage of xanthan gum is in salad dressing, but it is also used in baked goods, confectionery products, syrups, toppings, dry beverage mixes, frozen foods, and dairy products. Xanthan gum is synergistic with locust bean gum and guar gum, and they are often used together for enhanced gelation or viscosity.

We have surprisingly found that a high viscosity glucomannan: xanthan gum mixture has greatly increased satiety-promoting properties over those observed for glucomannan alone.

In the preferred embodiment, each serving comprises a mixture of glucomannan and xanthan gum at a weight ratio from about 1:1 to about 5:1. More preferably, each serving comprises glucomannan and xanthan gum at a weight ratio of about 4:1. Even more preferably, each serving comprises glucomannan and xanthan gum at a weight ratio of about 2: 0.5. Most preferably, the glucomannan: xanthan gum ratio is about 1.8: 0:44.

Preferably, glucomannan is present in the weight-loss supplement in an amount from about 0.1 g to about 5 g per serving. More preferably, the glucomannan is present in an amount from about 1 g to about 3 g per serving. Most preferably, the glucomannan is present in an amount of about 2 g per serving.

In the preferred embodiment, xanthan gum is present in an amount from about 0.1 g to about 5 g per serving. More preferably the xanthan gum is present in an amount from about 0.3 g to about 3 g per serving. Most preferably, the xanthan gum is present in an amount of about 0.44 g per serving.

In a preferred embodiment the thermogenic ingredients include caffeine, catechin-polyphenols, another methyl-xanthine and combinations thereof.

Preferably, the caffeine and catechin polyphenols are supplied in combination as a tea, green tea or as an enriched green tea extract.

Preferably a serving of the weight-loss supplement includes sufficient tea; green tea or as an enriched green tea extract to provide from about 25 to about 1000 mg of caffeine. More preferably, a serving of the weight-loss supplement includes sufficient green tea or enriched green tea extract to provide from about 50 to about 250 mg of caffeine. Most preferably, a serving of the weight-loss supplement includes sufficient green tea or enriched green tea extract to provide about 200 mg of caffeine.

Preferably a serving of the weight-loss supplement includes sufficient tea, green tea or enriched green tea extract to provide from about 1 to about 1000 mg of a catechin-polyphenol. More preferably, a serving comprises sufficient green tea or enriched green tea extract to provide from about 75 to about 500 mg of catechin-po1yphenol. Most preferably, a serving comprises sufficient green tea or enriched green tea extract to provide about 300 mg of BGCG.

Caffeine may alternatively be supplied as essentially pure caffeine or as an ingredient naturally occurring in other ingredients. Catechin-polyphenol may also be supplied as an essentially pure catechin-polyphenol or as an enriched catechin-polyphenol. An essentially pure or enriched catechin-polyphenol may be selected from the group consisting of epigallocatechin-gallate, epicatechin-gauate, epicatechin, catechin and epigallocatechin.

Optionally green tea is supplemented with essentially pure caffeine to supplement the thermogenic properties of green tea alone.

Optionally green tea is supplemented with an essentially pure catechin-polyphenol to supplement the thermogenic properties of green tea alone.

The present invention, according to one embodiment thereof, includes preferred active ingredients that aid in weight loss and help reduce side effects. For instance, the weight-loss supplement may, and preferably does, contain additional effective ingredients, which further enhance the weight loss effects of the supplement and aid in reducing side effects. The composition preferably further comprises one or more of the following ingredients: a chromium chelate, L-carnitine, Guarana, Yerba Mate, White Willow Bark, Garcinia Cambogia, Alpha Lipoic Acid, Ginseng, Gymnema Sylvestre and Cellulose. The chromium chelate is optionally selected from the group consisting of chromium picolinate and chromium polynicotinate. Ginseng is preferably American Ginseng.

A brief description of some of these additional effective ingredients and likely benefits is discussed in the following
**i.** Chromium chelate, for instance chromium picolinate, decreases insulin levels and improves glucose disposal.
**ii.** Acetyl-L-carnitine, a precursor of Acetyl-CoA in the tricarboxylic cycle, serves as a shuttle for fatty-acid transport into the mitochondria for beta oxidation (for energy production).
**iii.** Guarana (which may be supplied as seeds of Paullinia Cupana or an enriched extract) and Yerba Mate (which may be supplied as leaves of Ilex Paraguayensis or an enriched extract thereof) have several effects on the gastrointestinal system These include prolonging the digestive period and as satiety-promoting ingredients.
**iv.** White Willow Bark (Salix Alba) is a source of acetylsalicylic acid (the major component of aspirin) which has been observed to lower serum lipoprotein (a), Lp(a), a risk factor for developing atherosclerosis. Aspirin acts on Lp(a) by reducing apolipoprotein(a), gene transcription in those patients with elevated serum lipoprotein(a).
**v.** Garcinia cambogia has been shown to be a competitive inhibitor of the extra mitochondrial enzyme adenosine triphosphate-citrate lyase, which is a catalyst in extra mitochondrial cleavage of citrate to oxaloacetate and Acetyl-CoA which is required for the synthesis of fatty acids and lipogenesis in a high carbohydrate diet.
**vi.** Alpha Lipoic acid is an insulin modulator and a metabolic antioxidant that serves as protection against oxidative injury in non-neuronal and neuronal tissue.
**vii.** Gynmema Sylvestre

In a preferred embodiment, the weight-loss supplement includes Konjac Glucomannan at about 1.76g (e.g., 1.672g of Glucomannan - 95% purity); Xanthan gum at about 440 mg; Green Tea - Dry Leaf (4% caffeine, 45% EGCG) at about 667 mg (26.66mg Caffeine - 300 mg EGCG); Anhydrous Caffeine at about 173 mg; Guarana at about 1 mg; Yerba Mate at about 1 mg; Alpha Lipoic Acid at about 1 mg; and Chromium Polynicotinate to provide about 300 mcg of Elemental Chromium

The weight-loss supplement, according to one embodiment, may include: glucomannan at about 500 mg to about 5000 mg; xanthan gum at about 100 mg to about 1000 mg; sufficient green tea or green tea extract to provide caffeine at about 10 mg to about 600 mg, and catechin-polyphenol at about 45 to about 400 mg; chromium chelate which provides elemental chromium at a dose of about 10 mcg to about 500 mcg; L-carnitirie at about 1 mg to about 1000 mg; Guarana extract at about 50 mg to about 1004mg; yerba mate at about 1 mg to about 2000 mg; white willow bark at about 1 mg to about 500 mg; garcinia cambogia at about 1 mg to about 5000 mg; alpha lipoic acid at about 1 mg to about 1000 mg; ginseng at about 50 mg to about 500 mg; cellulose at about 1 mg to about 100 mg; and Gymnema Sylvestre at about 1 mg to about 1000 mg.

In one embodiment the weight-loss supplement includes: glucomannan, in a quantity of about 1.8g; xanthan gum, in a quantity of about 440 mg; enriched green tea extract (camellia sinensis), in a quantity of about 200mg; chromium chelate, in a quantity of about 0.3 mg; L-carnitine tartrate, in a quantity of about 100mg; guarana extract, in a quantity of about 530mg; alpha lipoic acid, 200mg; ginseng, in a quantity of about 330mg.

In an alternative embodiment the weight-loss supplement includes: glucomannan, in a quantity of about 1.8g; xanthan gum, in a quantity of about 440mg; enriched green tea extract (camellia sinensis), in a quantity of about 670 mg; chromium chelate, supplying about 0.3 mg of elemental chromium; L-carnitine, in a quantity of about 1mg; guarana, in a quantity of about 1 mg; yerba mate, in a quantity of about 1 mg; white willow bark, in a quantity of about 1 mg; garcinia cambogia, in a quantity of about 1mg; alpha lipoic acid, 1 mg; ginseng, in a quantity of about 1mg; and anhydrous caffeine, in a quantity of about 170 to 200 mg.

In an alternative embodiment the weight-loss supplement includes: Konjac in a quantity of about 1.76 g; Xanthan Gum in a quantity of about 440 mg; Green Tea (45% EGCG) in a quantity of about 500 mg; Green Tea (15% EGCG, 40% Caffeine) in a quantity of about 500 mg; Alpha Lipoic Acid in a quantity of about 100 mg; Guarana in a quantity of about 10 mg; Yerba Mate in a quantity of about 10 mg; Chromium Polynicotinate in a quantity of about 16.5 mcg.

In an alternative embodiment the weight-loss supplement includes: Oat Bran in a quantity of about 1.76 g; Xanthan Gum in a quantity of about 440 mg; Green Tea (45% EGCG) in a quantity of about 500 mg; Green Tea (15% EGCG, 40% Caffeine) in a quantity of about 500 mg Alpha Lipoic Acid in a quantity of about 100 mg; Guarana in a quantity of about 10 mg; and Yerba Mate in a quantity of about 10 mg.

The dosage form of the dietary supplement may be provided as a capsule, a caplet, a tablet and a ready-to-eat bar.

The dietary supplement compositions may be provided in accordance with customary processing techniques for herbal, dietary supplements wherein the active ingredients are suitably processed and encapsulated into cellulose capsules with suitable excipients.

The present invention also provides for a method for promoting weight loss including the steps of administering a weight-loss supplement that includes an effective amount of a satiety-promoting ingredient and an effective amount of a thermogenic ingredient, wherein the satiety-promoting ingredient is a dietary fiber and the thermogenic ingredient is selected from the group consisting of caffeine, catechin-polyphenol and combinations thereof. Preferably, the dietary fiber comprises a mixture of glucomannan and a gum. Preferably, the gum is selected from the group consisting of xanthan gum, guar gum, carageenan, and Locust bean gum. More preferably, the gum is xanthan gum Alternatively, the dietary fiber comprises a mixture of glucomannan and a blend of gums. Optionally, the blend of gums comprises at least one compound selected from the group consisting of xanthan gum, guar gum, carageenan, and locust bean gum

In a preferred embodiment, the directions (for both men and women) for use of the weight-loss supplement is as follows: as a dietary supplement, it is advised that users take 4 capsules with 12-ounce glass of water 3 times daily, approximately 30 to 60 minutes before meals (preferably before breakfast, lunch, and diner) To assess individual tolerance, it is advised that users follow the dosing chart below.

| | |
|---|---|
| Day 1 to Day 3 | 2 capsules, 3x daily |
| Day 4 to Day 7 | 3 capsules, 3x daily |
| Day 8 & beyond | 4 capsules, 3x daily |

For best results, it is advised that users combine DIET-TBCH with a calorie-reduced diet and an exercise program It is also advised that users do not snack after dinner, that they consume ten 12-ounce glasses of water per day.

The weight-loss supplement is optionally administered at meals. Preferably, the weight-loss supplement is administered three times a day. More preferably, the weight-loss supplement is administered three times a day for a period of at least about a week Even more preferably, the weight-loss supplement is administered three times a day for a period of about at least 4 weeks. Most preferably, the weight-loss supplement is administered three times a day for a period of about at least 8 weeks.

The present invention also provides for a method of making a weight-loss supplement In accordance with one embodiment, the method includes the steps of premixing a chromium chelate with microcrystalline cellulose; adding the following ingredients to the premix: Konjac Glucomannan, Xanthan Gum, Green Tea - Dry Leaf, Anhydrous Caffeine, Guarana, Yerba Mate, Alpha-lipoic acid; and in an additional step adding the following ingredients Magnesium Stearate and silica which had been pre-sifted; blending and mixing for 30 minutes. Finally, checking for uniformity/homogeneity and then aliquoting into a serving.

Although the following examples illustrate the practice of the present invention in some of its embodiments, the examples should not be construed as limiting the scope of the invention. Other embodiments will be apparent to one skilled in the art from consideration of the specification and examples.

### EXAMPLES

### EXAMPLE 1

A weight-loss supplement including the following ingredients per serving was prepared.

| Component | Amount Per Serving |
|---|---|
| Glucomannan | 1.76g |
| Xanthan gum | 440mg |
| Enriched Green Tea (camellia sinensis) | 200mg |
| Chromium poly-nicotinate | 0.300 mg |
| L-carnitine tartrate | 100mg |
| Guarana | 528mg |
| Alpha lipoic acid | 200 mg |
| American Ginseng | 330mg |

### EXAMPLE 2

A weight-loss supplement including the following ingredients per serving was prepared.

| Component | Amount Per Serving |
|---|---|
| Glucomannan | 1.76g |
| Xanthan gum | 440 mg |
| Caffeine | 200mg (173mg green tea and 27mg anhydrous caffeine) |
| Catechin-polyphenol | 300 mg of BGCG |
| Elemental Chromium as Chromium picolinate | 0.3 mg |
| L-carnitine | 1 mg |
| Guarana | 1 mg |
| Yerba mate | 1 mg |
| White willow bark | 1 mg |
| Garcinia cambogia | 1 mg |
| Alpha lipoic acid | 1 mg |

### Example 3

A weight-loss supplement comprising the following ingredients is prepared as capsules according to the following formulation.

| ***Active Ingredients*** | **g/serv** | **g/eapsule** | **Formula %** | **Actives per Serving** |
|---|---|---|---|---|
| Konjac Glucomanan | 1.760 | 0.440 | 57.789 | 1.672 g Glucomannan - 95% purity |
| Xanthan Gum | 0.440 | 0.116 | 14.447 | 440 mg Xantan Gum |
| Green Tea - Dry leaf (4% Caffeine; 45% EGCG) | 0.667 | 0.167 | 21.888 | 26.66 mg Caffeine - 300 mg EGCG |
| Anhydrous Caffeine - USP grade | 0.173 | 0.043 | 5.679 | 173 mg USP grade Caffeine |
| Guarana - Dry seed | 0.001 | 0.0003 | 0.033 | 1 mg Guarana |
| Yerba Mate - Dry Leaf | 0.001 | 0.0003 | 0.033 | 1 mg Yerba Mate |
| Alpha-lipoic acid | 0.001 | 0.0003 | 0.033 | 1 mg Alpha-Lipoic Acid |
| Chromium Polynicotinate | 0.00300 | 0.00075 | 0.099 | 300 meg Elemental Chromium |

The directions for use of the weight-loss supplement is as follows

**DIRECTION FOR MEN & WOMEN: As a dietary supplement, take 4 capsules with 12-ounce glass of water 3 times daily, approximately 30 to 60 minutes before meals (preferably before breakfast, lunch, and dinner). To access individual tolerance, follow the dosing chart below.**

| | | |
|---|---|---|
| Day 1 to Day 3 | 2 capsules, 3x daily | For best results, combine DIET-TECH with a calorie-reduced diet and an exercise program Do not snack after dinner. Consume ten 12-ounce glasses of water per day. Read the entire label use and follow directions. |
| Day 4 to Day 7 | 3 capsules, 3x daily | |
| Day 8 & beyond | 4 capsules, 3x daily | |

### EXAMPLE 4: Manufacturing the weight-loss supplement

1. PRBMTX: Chromium Chelate and microcrystalline cellulose (MCC) 102 were premixed separately for 10 minutes.
2. Add the following ingredients to the premix from step 1: Konjac Glucomannan, xanthan Gum, Green Tea - Dry Leaf, Anhydrous Caffeine, Guarana, Yerba Mate, Alpha-lipoic acid and the remaining MCC 102 were sifted through a mesh #10, and then added into the mixer and mixed for 60 minutes.
3. Magnesium Stearate and silica were pre-sifted through mesh #30 and added to the mixture from step 2 and blended and mixed for 30 minutes.
4. The product was checked for uniformity/homogeneity.
5. The product was then aliquoted into an encapsulated serving.

### EXAMPLE 5:

### A randomized double-blind placebo-controlled research study of the administration a formulation.

A clinical study was performed which demonstrated that individuals administered a formulation comprising glucomannan, xanthan gum, caffeine and catechin-polyphenol (the "Active Product Group"), would achieve a greater reduction in body weight when compared to a group consuming a placebo product (the "Placebo Product Group") for the same period of time. The formulation per serving used in the clinical trial ("formulation A") was as follows: chromium picolinate = 300 micrograms, i.e. 0.3 mg; L-carnitine = 1 mg; green tea = 666.6 mg (supplying 300 mg epicallocatechingallate and 26.66 mg caffeine); guarana = 1 mg (supplying 0.36 mg caffeine); -yerba mate = 1 mg (supplying 0.01 mg caffeine); glucomannan-xanthan gum blend (80:20 ratio) = 2.2 g; white willow bark = 1 mg; garcinia cambogia = 1 mg (supplying 0.55 mg Hydroxycitric acid); alpha lipoic acid = 1 mg and caffeine anhydrous = 172.97 mg. The formulation used in Examples 3 and 5 was formulation A.

**Methods:** Of 95 patients screened by telephone, 54 were enrolled; all diagnosed with being overweight. They were randomized into two treatment groups: the Active Product Group who were administered the formulation A and Placebo Product Group, to whom a placebo of gelatin capsules comprising essentially cellulose was administered. Both the Active Product and Placebo Product Groups took their respective product three times daily (four capsules per serving) prior to each meal for eight weeks. Both groups reduced their caloric intake by 500 kcal below the level required to maintain their ideal body weight per day, based on guidelines set by a dietician.

**Results:** After eight weeks, the Active Product Group lost an average of 10.1 lb while the Placebo Product Group lost an average of 5.5 lb (p=0.004). Body Mass Index (BMI) was reduced by 1.7 for the Active Group compared to 0.9 for the Placebo Group (p=0.02). The average loss off of the waist in the Active Group was 2.7 inches compared to 1.4 inches in the Placebo Group (p=0.04). The average loss off of the thigh was 1.4 inches for the Active Group compared to 0.3 inches for the Placebo Group (p=0.03). Also, there were no significant differences between the two treatment groups for change in percent body fat, fat mass, fat-free mass, heart rate, systolic and diastolic blood pressure, and hip or arm measurements.

The purpose of this randomized double-blind placebo-controlled research study was to assess the effectiveness of the formulation, in reducing body weight and percent body fat in overweight males and females (19 to 45 yo) with BMI ≥30 (kg/m²) or body fat ≥ 25%:

### Example 6:

### A randomized double-blind placebo-controlled research study of the administration of a weight loss formulation.

Study Design: A total of 66 overweight males and females, 19 to 45 years old, with BMI ≥ 30 were selected from those responding to announcements in the newspaper, at local colleges or in private practice clinics. Eligibility was assessed through telephone pre-screening. The purpose of the study was fully explained to potential participants. During the initial visit to the study site, the Informed Consent and Bill of Rights forms were thoroughly reviewed with subjects by the Principal Investigator (PI) or Clinical Research Coordinator (CRC) prior to beginning the study. Subjects were then instructed by the PI or CRC to read and sign the Informed Consent and Bill of Rights forms, if agreeable. Financial compensation was discussed ($150.00). Subjects were also required to complete a screening health questionnaire and undergo a limited physical examination by the PI. Twelve of the 66 enrolled in the study were disqualified per PI due to poor electrocardiogram (EKG). Seven of the 54 enrolled subjects were disqualified per sponsor due to Thanksgiving. Forty-seven subjects were fnally enrolled in the study. Also, during the study period,15 enrolled subjects withdrew from the study due to personal reasons, side effects, or work schedule conflicts. Therefore, 32 subjects completed the study (Active Group= 14; Placebo Group= 18).

To be included, subjects had to be diagnosed as being overweight (per PI), with BMI ≥ 30 kg/m² or body fat ≥ 25% (determined by a Bioelectric Impedance Analysis), 19 to 45 years old, and all subjects agreed to stay on a low-calorie diet (agreeing to adjust their caloric intake to 500 kcal below that required to maintain their ideal body weight per day based on dietician guidelines). Subjects were excluded for the following reasons: 1) they did not pass the physical exam per the PI; 2) they had any metabolic disorder including known electrolyte abnormalities; 3) they had heart disease, arrhythmias, diabetes, thyroid disease or hypogonadism, a history of hypertension, hepatorenal, musculoskeletal, autoimmune, or neurologic disease; 4) they were taking thyroid, hyperlipidemic, hypoglycemic, anti-hypertensive, anti-depressant, or androgenic medications; 5) they had taken nutritional supplements that may affect muscle mass (e.g., creatine, β-hydroxy β-methylbutyrate monohydrate (HMB) or diet products (such as those containing ephedra) within six months of the start of the study; 6) they were currently using any medication that conflicted with the product ingredients; 7) they were pregnant, lactating or women not taking medically approved birth control; 8) women planning to become pregnant within 30 days of the start of the study; or, 9) they were habitual smokers.

A urine pregnancy test was administered, and contraceptive method form was read and signed by female subjects. All subjects were asked to tum in a personal three-day diet record (pre-treatment only), to the dietician prior to the beginning of the study.

Bioelectrical Impedance Analysis (BIA) was used to measure bodyweight, BMI and changes in body composition (fat mass, fat percentage) at 0, 2, 4, 6, and 8 weeks. Vital signs were also taken at 0, 2, 4, 6, and 8 weeks. Other measures of body composition were assessed by Anthropometry (circumference measures were used to determine waist-to-hip ratios, waist-to-thigh ratios and waist-to-arm ratios at weeks 0 and 8).
**BMI, BIA and Anthropometry measures**. Clinicians use BMI, BIA and Anthropometry to measure body weight or to assess if a patient is overweight or obese. The most common method is BMI, a measure of body fat based on height and weight that applies to both adult men and women. It is a mathematical formula that measures a person's body mass by dividing a person's weight in kg by the square of his or her height in meters (BMI = kg/m²). Individuals with a BMI 25 to 29.9 are considered overweight. An individual with a BMI equal to or greater than 30 is considered obese.

Another method for diagnosing overweight or obesity is called Bioelectrical Impedance Analysis (BIA). It measures changes in body composition. This is carried out using a precision scale with electrodes in foot sensor pads that conduct a safe current through the body. A constant small current of 800 uA at a fixed frequency (50 kHz) is passed through these electrodes. The voltage drop between these electrodes serves as a measure of impedance or resistance. The person steps on the scale. His/her height, age and gender are entered into a computer. Weight and body fat content are calculated in less than a minute. BIA measures the opposition (impedance), to the flow of the electric current through body fluids contained in lean and fat tissue. Opposition to flow of the electric current (impedance) is low in lean tissue. Intracellular fluid and electrolytes are primarily contained in lean tissue. Impedance is high in fat tissue and proportional to total body water volume.

Anthropometry is used to measure overweight and obesity as well. It measures the variation in physical dimensions and body composition. Circumference measures are carried out to determine waist-to-hip ratios, waist-to-thigh ratios and waist-to-arm ratios.
**Randomization.** The eight-week randomized double-blind placebo-controlled research study was conducted on 54 randomized subjects who met inclusion criteria. Subjects were randomly divided into groups. The Active Product Group consisted of individuals who received formulation A, *see supra*: (n= 27), and the Placebo Product Group (n=27) who received essentially gelatin capsules consisting of cellulose. See Table 1 for demographics.

**Pre-study dietary assessment, dietary instruction and study products**. Subjects were provided with three-day pre-treatment diet records log, dietary log, product administration instruction sheet and products. Subjects (both groups) were given a nutritional counseling session to discuss dietary modification and management. Both groups reduced their caloric intake by 500 kcal below that required to maintain their ideal body weight per day based on dietician guidelines. Based on their diets and food preference, a dietary plan was prepared for them by a dietician. Subjects of both groups took the active or placebo product three times daily (four capsules per serving) prior to each meal for eight weeks.

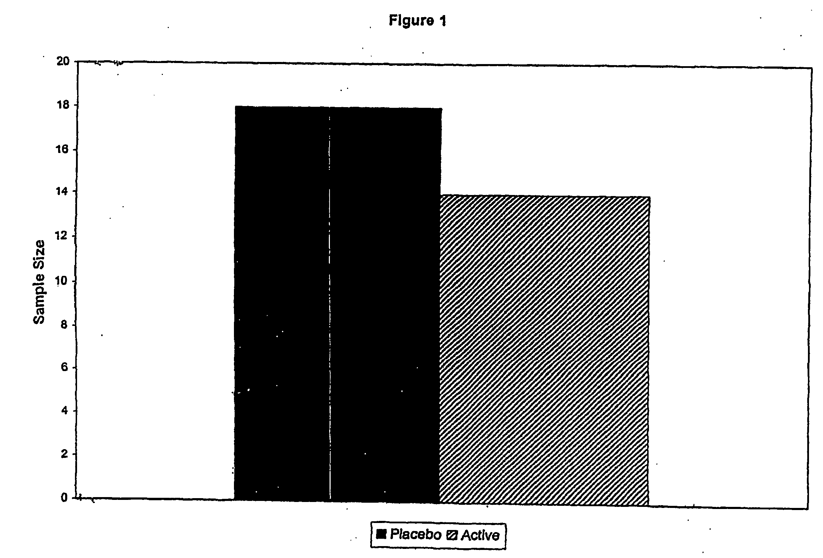

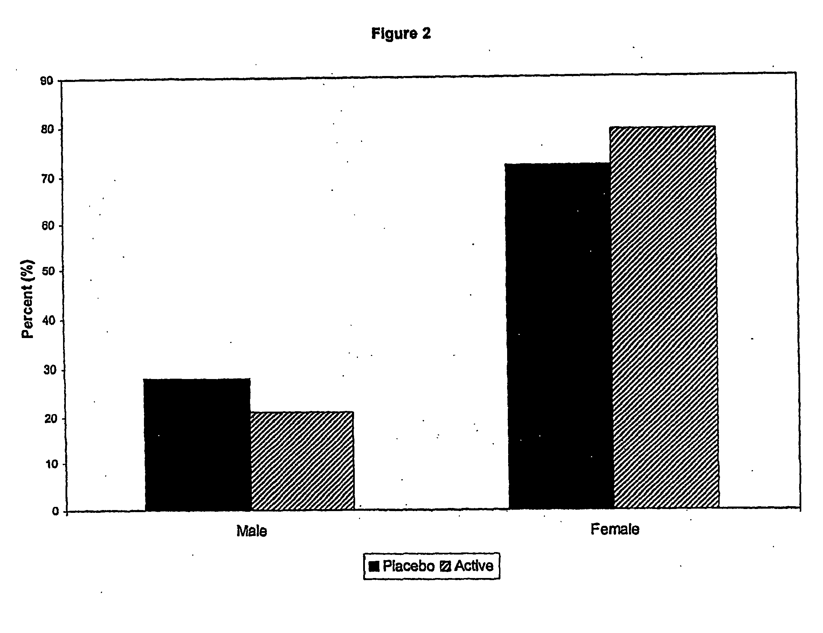

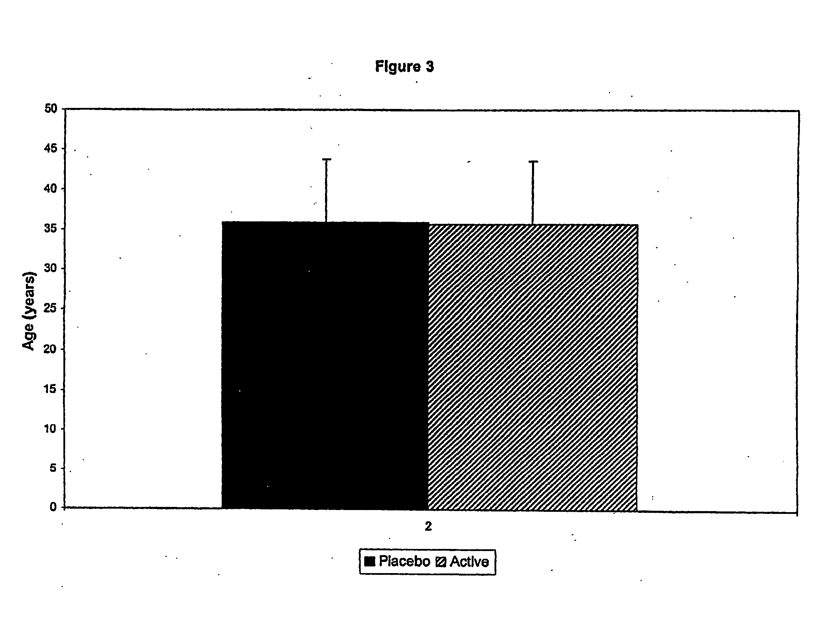

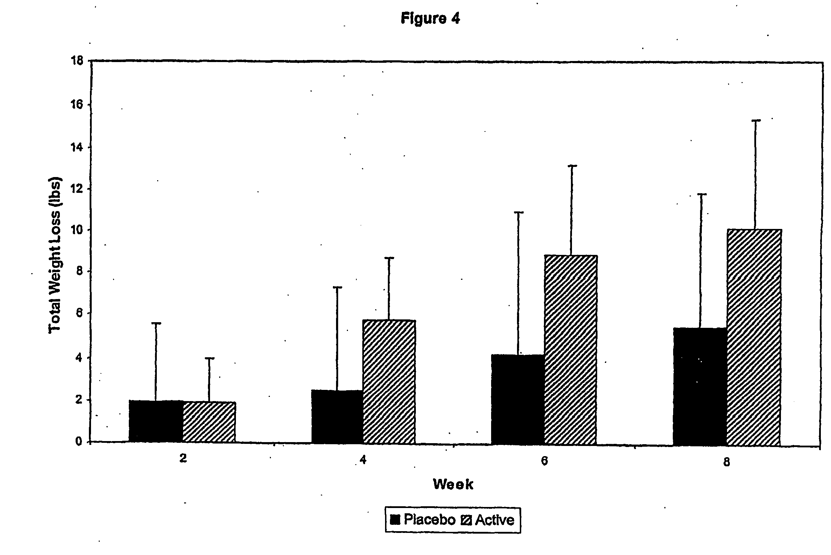

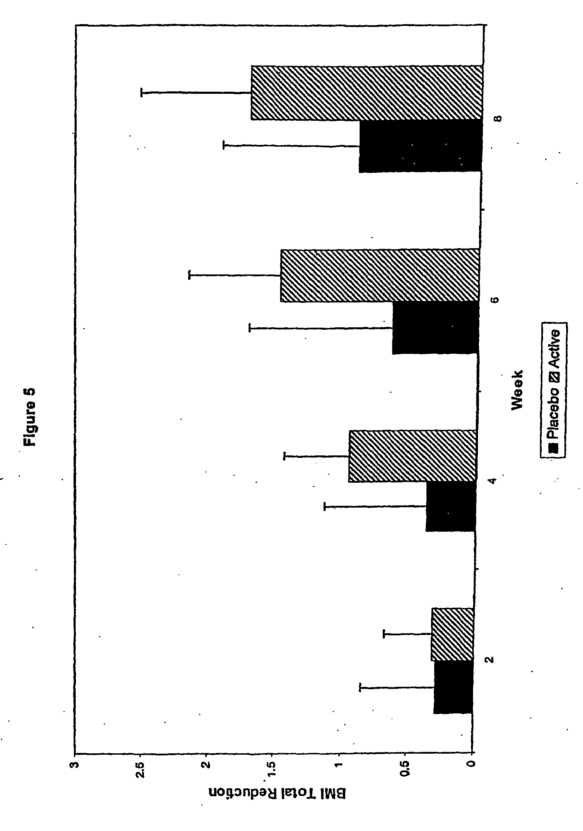

These ingredients have been observed to be effective in treating weight loss. Compliance was monitored by having the study coordinator call each patient twice per week to discuss their symptoms and review the side effects. The CRC also dispensed the products in different intervals, coordinated proper subject selection and provided education, management and encouragement. Any side effects or symptoms due to the product were also discussed and noted by the CRC.
**Statistical Methods.** Means and standard deviations were reported for continuous variables and frequencies and percentages were reported for categorical variables. Independent samples t-tests were used to compare differences between groups for continuous variables and Fisher's Exact tests were used to compare categorical variables. The accepted level of significance for all tests was alpha=0.05. The SAS Institute, Cary, North Carolina, was used for all analyses. **Randomization and comparison of completers versus dropouts.** A total of 54 subjects were randomized, 27 (50%) to each treatment group (Placebo and Active). Twenty-two dropped out of the study {13 (48%) Active vs. 9 (33%) Placebo, p=0.41}. The subjects who dropped out were younger (28.4 years) than those subjects who completed the study (35.8 years) (p=0.0009). There were no differences in gender distribution between those subjects who dropped out and those who completed the study (see table 2).
**Demographics.** Thirteen in the Active Product Group and 18 in the Placebo Product Group completed the study (see Fig. 1). The average age (sd) for subjects who completed the study was 35.6 (7.8) Active and 35.9 (7.7) Placebo (p=0.91) (see Table 1 and Fig. 3). Seventy-nine percent of the active subjects and 72% of the placebo subjects were female (p=0.21) (see figure 2).
**Follow-up.** Presented in Tables 3a and 3b are week 0 (baseline), week 8 and change at week 8 (week 8 measure - week 0 measure) with measures stratified by treatment group. There were significant differences between the two treatment groups after eight weeks for total weight loss, reduction in BMI, and total inches lost off of waist and thigh measurements. After eight weeks, the Active Product Group lost an average of 10.1 lb (1.3 lb per week) while the Placebo Product Group lost 5.51b (0.7 lb per week) (p=0.004) (see figure 4). BMI was reduced by 1.7 for the Active Product Group compared to 0.9 for the Placebo Product Group (p=0.02) (see figure 5). The average loss off of the waist in the Active Product Group was 2.7 inches compared to 1.4 inches in the Placebo Product Group (p=0.04). The average loss off of the thigh was 1.4 inches for the Active Product Group compared to 0.3 inches for the Placebo Product Group (p=0.03). There were no significant differences between the treatment groups for change in percent body fat, fat mass, fat free mass, heart rate, systolic and diastolic blood pressure, and hip and arm measurements (p>0.05).
**Self-reported side effects.** Table 4 presents the participant self-reported side-effects. A similar proportion (64% active, 50% placebo) in each group reported having one or more side effects (p=0.49). More subjects in the Active Product Group (43%) reported a decreased appetite than in the Placebo Product Group (6%) (p=0.03). There were no significant differences between the two treatment groups for any of the other self-reported side effects.

This eight-week randomized double-blind placebo-controlled research study has shown that administration of the formulation A resulted in greater reduction in body weight, BMI, decrease in appetite, as well as an increase in the amount of inches lost off of waist and thigh measurements when compared to the Placebo Product Group. The study was conducted on 54 randomized overweight/obese individuals meeting inclusion criteria, ages 19-45 years old with BMI ≥ 30 kg/m² or body fat ≥ 25%.

Based on statistical analysis results, the formulation A has been shown to be significantly effective in reducing body weight, BMI, appetite, as well as increasing the amount of inches lost off of waist and thigh measurements. There were no significant differences between the two treatment groups for change in percent body fat, fat mass, fat free mass, heart rate, systolic and diastolic blood pressure, and hip or arm measurements (p>0.05). A similar proportion of side effects were reported in both the Active Group and the Placebo Group.

**Table 1:**

| Sample size and demographic comparisons between treatment groups | | | |
|---|---|---|---|
| | **Active** | **Placebo** | **p-value¹** |
| Number randomized | 27 (50%) | 27 (50%) | |
| Dropouts | 13 (48%) | 9 (33%) | 0.41^{F} |
| Gender | | | 1.00 |
| Male | 3 (21%) | 5 (28%) | |
| Female | 11 (79%) | 13 (72%) | |
| Age, years | 35.6 (7.8)² | 35.9 (7.7) | 0.91^{T} |
| Height (inches) | 64.7 (3.1) | 66.4 (4.3) | 0.21 |

| | | | |
|---|---|---|---|
| ¹ p-values: F=Fisher's Exact test, T=T-test ² Mean (sd) | | | |

**Table 2:**

| Dropouts vs. Completers | | | |
|---|---|---|---|
| | **Dropouts** | **Completed Study** | **p-value¹** |
| Sample size | 22 (41%) | 32 (59%) | |
| Age, years | 28.4 (7.6)² | 35.8 (7.6) | 0.0009^{T} |
| Gender | | | 0.74^{F} |
| Female | 82% | 75% | |
| Male | 18% | 25% | |

| | | | |
|---|---|---|---|
| ¹ p-values: F=Fisher's Exact test, T=T-test ² Mean (sd) | | | |

**Table 3a:**

| Comparison of baseline, follow-up and change³ of weight, BMI and bioelectrical impedance analysis (BIA) measures between treatment groups. | | | |
|---|---|---|---|
| | **active** **(n=14)** | **Placebo** **(n=18)** | **p-value¹** |
| Weight (1b) | | | |
| Week 0 | 207.3 (42.7)² | 208.5 (42.5) | 0.94 |
| Week 8 | 197.2 (37.9) | 203.0 (39.3) | 0.68 |
| Change³ | -10.1 (5.2) | -5.5:(6.3) | 0.03 |
| | | | |
| BMI | | | |
| Week 0 | 34.8 (6.2) | 33.4 (7.1) | 0.58 |
| Week 8 | 33.1 (5.5) | 32.6 (6.5) | 0.80 |
| Change³ | -1.7 (0.8) | -0.9 (1.0) | 0.02 |
| | | | |
| % Body Fat | | | |
| Week 0 | 40.8 (8.9) | 40.2 (7.2) | 0.82 |
| Week 8 | 41.2 (9.9) | 40.3 (8.2) | 0.77 |
| Change³ | 0.4 (2.3) | 0.1 (2,9) | 0.74 |
| | | | |
| Fat Mass | | | |
| Week 0 | 86.8 (33.7) | 84.8 (29.7) | 0.86 |
| Week 8 | 8334 (32.9) | 83.2 (30.6) | 0.99 |
| Change³ | -3.4 (5.0) | -1.6 (8.2) | 0.47 |
| | | | |
| Fat Free Mass | | | |
| Week 0 | 120.6 (20.2) | 123.7 (24.3) | 0.70 |
| Week 8 | 113.8 (18.8) | 119.8 (20.6) | 0.41 |
| Change³ | -6.7 (5.3) | -3.9 (7.5) | 0.24 |

| | | | |
|---|---|---|---|
| ¹ T-test p-values ² Mean (sd) ³ Change = week 8 value - week 0 value | | | |

**Table 3b:**

| Comparison of baseline, follow-up and change³ for resting heart rate, blood pressure and body measurements between treatment groups. | | | |
|---|---|---|---|
| | **Active** **(n=14)** | **Placebo** **(n-18)** | **p-value¹** |
| Heart rate | | | |
| Week 0 | 75.2 (8.2) | 71.9 (8.5) | 0.28 |
| Week 8 | 77.4 (4.1) | 72.3 (6.6) | 0.02 |
| Change³ | 2.2 (7.2) | 0.4 (5.5) | 0.42 |
| | | | |
| Systolic BP | | | |
| Week 0 | 114.6 (8.3) | 114.3 (8.8) | 0.94 |
| Week 8 | 114.4 (6.6) | 113.2 (4.9) | 0.56 |
| Change³ | -0.1 (5.7) | -1.1 (8.9) | 0.73 |
| | | | |
| Diastolic BP | | | |
| Week 0 | 75.6 (10.4) | 72.4 (9.0) | 0.37 |
| Week 8 | 76.0 (4.7) | 74.6 (6.7) | 0.50 |
| Change³ | 0.4 (10.4) | 2.1 (7.7) | 0.60 |
| | | | |
| Waist (inches) | | | |
| Week 0 | 40.4 (5.5) | 40.0 (4.9) | 0.85 |
| Week 8 | 37.6 (5.2) | 38.6 (5.5) | 0.62 |
| Change³ | -2.7 (1.3) | -1.4 (2.1) | 0.04 |
| | | | |
| Hip (inches) | | | |
| Week 0 | 47.2 (6.0) | 46.6 (5.7) | 0.76 |
| Week 8 | 45.1 (6.0) | 45.8 (6.3) | 0.74 |
| Change³ | -2.1 (1.9) | -0.8 (2.4) | 0.10 |
| | | | |
| Thigh (inches) | | | |
| Week 0 | 24.8 (3.3) | 23.6 (2.6) | 0.28 |
| Week 8 | 23.4 (2.8) | 23.4 (2.3) | 0.97 |
| Change³ | -1.4(1.3) | -0.3 (1.3) | 0.03 |
| | | | |
| Arm (inches) | | | |
| Week 0 | 13.5 (1.4) | 13.4 (1.5) | 0.83 |
| Week 8 | 12.6 (1.1) | 12.7 (1.4) | 0.96 |
| Change³ | -0.9 (0.6) | -0.7 (0.5) | 0.51 |

| | | | |
|---|---|---|---|
| ¹ T-test p-values ² Mean (sd) ³ Change = week 8 value - week 0 value | | | |

**Table 4:**

| Self Reported Side Effects | | | |
|---|---|---|---|
| | **Active** **(n=14)** | **Placebo** **(n=18)** | **Fisher's Exact** **p-value** |
| Subjects with one or more side effects | 9 (64%) | 9 (50%) | 0.49 |
| Increased urine | 1 (7%) | 2(11%) | 1.00 |
| Increased appetite | 0 | 3 (17%) | 0.24 |
| Aggressiveness | 0 | 2 (11%) | 0.49 |
| Restlessness | 0 | 3 (17%) | 0.24 |
| Irritability | 0 | 2(11%) | 0.49 |
| Dizziness | 0 | 1 (6%) | 1.00 |
| Insomnia | 0 | 1 (6%) | 1.00 |
| Constipation | 1 (7%) | 2 (11%) | 1.00 |
| Anxiety | 1 (7%) | 2 (11%) | 1.00 |
| Headache | 1 (7%) | 5 (28%) | 0.20 |
| Increased sweating | 1 (7%) | 1 (6%) | 1.00 |
| Tremors | 1 (7%) | 1 (6%) | 1.00 |
| Vomiting | 1 (7%) | 0 | 0.44 |
| Dry mouth | 3 (21%) | 2 (11%) | 0.63 |
| Decreased appetite | 6 (43%) | 1 (6%) | 0.03 |
| Heartburn | 1 (7%) | 0 | 0.44 |
| Sleepy | 0 | 1 (6%) | 1.00 |

### EXAMPLE 7:

The effect of a combination of caffeine and BGCG on metabolic rate was examined in a clinical trial.

The energy expenditure of 15 subjects was examined in a metabolic chamber used to calculate metabolic rate in free-living subjects. Metabolic rate is calculated in the chamber via indirect calorimetry; the validity and reproducibility of the measurements of metabolic rate are documented in White et al. (White, M., G. Bouchard, B. Bueman, N. Almeras, J. Despres, C. Bouchard, and A. Tremblay. Reproducbility of 24-h energy expenditure and macronutrient oxidation rates in an indirect calorimeter. J. Appl. Physiol. 80:133-139, 1996). While in the metabolic chamber for 24 hours the subjects were fed a controlled diet with a caloric content calculated to be equivalent to their resting metabolic rate (RMR), the energy expended by the subjects at rest. The RMR was determined separately during a preliminary trial. The same diet was fed to the subjects on each trial. The subjects sat and/or rested in the chamber during their 24-hour trial.

Bach subject was administered their treatment which was contained in two capsules comprising either placebo or 200 mg of caffeine and variable amounts of BGCG at the following times on each trial: at 0800 h, followed by the standard breakfast 30 minutes later; at 1200 h followed by the standard lunch 30 minutes later, and, at 1600 h followed by a standard dinner 30 minutes later.

As shown in Table 5 the energy expenditure of resting subjects, as measured in the metabolic chamber, was significantly higher for individuals when they were administered caffeine and EGCG as compared to placebo trial (p<0.05). Bach of the trials was significantly different from the placebo trial but there was no statistically significant difference among the various EGCG trials.

**Table 5**

| 24 Hour Energy Expenditure (kcaI) | | | | | |
|---|---|---|---|---|---|
| **Composition Administered** | **Placebo** | **90 mg EGCG** **+ 200 mg Caffeine** | **200 mg EGCG** **+ 200 mg Caffeine** | **300 mg EGCG** **+ 200 mg Caffeine** | **400 mg EGCG** **+ 200 mg Caffeine** |
| **Mean Values for 15 subjects** | **2252** | **2410** | **2422** | **2433** | **2450** |

### EXAMPLE 8

A weight-loss supplement including the following ingredients per serving was prepared.

| | **Amount per Serving** |
|---|---|
| Konjac | 1.76 g |
| Xanthan Gum | 440 mg |
| Green Tea (45% BGCG) | 500 mg |
| Green Tea (15% BGCG, 40% Caffeine) | 500 mg |
| Alpha Lipoic Acid | 100 mg |
| Guarana | 10 mg |
| Yerba Mate | 10 mg |
| Chromium Polynicotinate | 16.5 mcg |

### EXAMPLE 9

A weight-loss supplement including the following ingredients per serving was prepared.

| **Amount per Serving** | |
|---|---|
| Oat Bran | 1.76 g |
| Xanthan Gum | 440 mg |
| Green Tea (45% EGCG) | 500 mg |
| Green Tea (15% BGCG, 40% Caffeine) | 500 mg |
| Alpha Lipoic Acid | 100 mg |
| Guarana | 10 mg |
| Yerba Mate | 10 mg |

## Claims

1. A weight-loss supplement comprising:
(a) a satiety-promoting ingredient that includes a dietary fiber comprising oat bran and;
(b) a thermogenic ingredient selected from the group consisting of caffeine, catechin-polyphenol and combinations thereof.

2. The supplement of claim 1, further comprising a gum.

3. The supplement of claim 2, wherein the gum is selected from the group consisting of xanthan gum, guar gum, cargeenan, and locust bean gum.

4. The supplement of claim 1, 2 or 3, comprising:
Oat Bran, in a quantity of about 1. 8 mg ;
Xanthan gum, in a quantity of about 440 mg ;
Green Tea (45% EGCG), in a quantity of about 500 mg;
Green Tea (15% EGCG, 40% Caffeine) in a quantity of about 500 mg;
Guarana, in a quantity of about 10 mg ;
Yerba Mate, in a quantity of about 10 mg; and
Alpha Lipoic Acid, in a quantity of about 100 mg.

5. The supplement of one of the preceeding claims, being comprised in a tablet, a caplet, a ready-to-eat dietary supplement or food bar.

6. The supplement of one of the preceeding claims, wherein a serving comprises from about 25 mg to about 1000 mg of caffeine.

7. A method for inducing non-therapeutic weight loss that comprises the step of:
administering an effective amount of a weight-loss supplement that comprises a satiety-promoting ingredient that includes a dietary fiber comprising oat bran and a thermogenic ingredient selected from the group consisting of caffeine, catechin-polyphenol and combinations thereof.

8. The method of claim 7, wherein the weight-loss supplement is administered about ½ to 1 hour before meals.

9. The method of claim 7 or 8, wherein the weight-loss supplement is administered three times a day.

10. The method of claim 7, 8 and 9, wherein the weight-loss supplement is administered three times a day for a period of at least about a week.

11. The method of one of the claims 7 through 10, wherein the supplement is administered as a tablet, a caplet, a ready-to- eat dietary supplement or food bar.

12. The method of one of the claims 7 through 11, wherein the supplement further comprises a gum.

13. The method of claim 12, wherein the gum is selected from the group consisting of xanthan gum, guar gum, cargeenan, and locust bean gum.

14. The method of one of the claims 7 through 13, wherein a serving comprises from about 25 mg to about 1000 mg of caffeine.
